# EUROPÄISCHE PATENTSCHRIFT

(11) **EP 2 298 259 B1**
(45) Veröffentlichungstag und Bekanntmachung des Hinweises auf die Patenterteilung: **23.01.2013**
(21) Anmeldenummer: 10009402.8
(22) Anmeldetag: 09.09.2010
(51) Int. Cl.: A61F 13/20

(54) **Vorrichtung zum Herstellen eines Tampons**
Device for manufacturing of a tampon
Dispositif de fabrication d'un tampon

(30) Priorität: 09.09.2009 DE 202009012237 U
(43) Veröffentlichungstag der Anmeldung: 23.03.2011
(60) Teilanmeldung: 11007729.4
(73) Patentinhaber: Rauscher Consumer Products GmbH, 1140 Wien (AU)
(72) Erfinder: Steinlechner, Erik, 2500 Baden (AT); Pötsch, Ernst, 2525 Schönau (AT); Mikulaschek, Marlene Sophie, 1230 Wien (AT); Posch, Karl-Heinz, 2801 Katzelsdorf (AT); Süssle, Wolfgang, 2500 Baden (AT)
(74) Vertreter: Leinweber & Zimmermann

(56) Entgegenhaltungen:
- GB-A- 2 056 283
- GB-A- 2 073 592
- US-A- 2 386 590
- US-A- 3 343 225
- US-A- 4 018 225
- US-A- 5 634 248
- US-A1- 2004 030 280

## Beschreibung

Die Erfindung betrifft eine Vorrichtung zum Herstellen eines Tampons zum Einführen in eine Körperhöhle, insbesondere in die weibliche Scheide, mit einem im wesentlichen zylindrischen Saugkörper, der eine sich in Richtung auf ein axiales Ende davon verjüngende Kuppe aufweist.

Tampons der gerade beschriebenen Art werden zur Aufnahme von Körperflüssigkeit in der Wundversorgung, maßgeblich aber im Zusammenhang mit der weiblichen Menstruation eingesetzt. Dabei trifft die Menstruationsflüssigkeit von außen auf den Tampon und dringt von außen nach innen in den Saugkörper ein. Demnach gelangt die Flüssigkeit durch die äußeren Tamponschichten in den üblicherweise verdichteten Tamponkern, welcher das größte Absorptionsvermögen aufweist. Allerdings besteht bei der Verwendung herkömmlicher Tampons das Risiko, daß die äußeren Schichten des Tampons bereits mit Flüssigkeit gesättigt sind, bevor die Flüssigkeit zum Tamponkern vordringt und dann die Gefahr des Auslaufens von Flüssigkeit auftritt, obwohl der Saugkern des Saugkörpers noch nicht mit Flüssigkeit gesättigt bzw. vollständig benutzt ist.

Zur Behebung dieses Mangels wurden bereits Tampons vorgeschlagen, deren Saugkörper sich parallel zur Zylinderachse erstreckende Rillen in ihrer äußeren Begrenzungsfläche aufweisen. Zur Verbesserung der Absorptionseigenschaften von Tampons wurde auch vorgeschlagen, diese Rillen wellenförmig und/oder wendelförmig die Zylinderachse des im wesentlichen zylindrischen Saugkörpers umlaufend auszuführen.

Wenngleich mit entsprechend ausgeführten Tampons eine Verbesserung der Absorptionseigenschaften erreicht werden kann, hat es sich gezeigt, daß weiterhin das Auslaufen von Körperflüssigkeit aus der mit dem Tampon zu verschließenden Körperhöhle beobachtet wird, obwohl das Absorptionsvermögen des Tampons noch nicht vollständig ausgenutzt ist.

In der US 4,318,405 ist ein Tampon mit einer sich in Richtung auf ein axiales Ende verjüngenden Kuppe beschrieben, bei dem eine Kapsel zum Einbringen pharmazeutisch wirksamer Substanzen in eine Vertiefung der Kuppe eingesetzt ist. Mit diesen Tampons können therapeutisch wirksame Substanzen eingebracht werden. Eine Verbesserung im Hinblick auf die Aufnahme von aus der Körperhöhle auslaufender Körperflüssigkeit wird durch Einsatz dieser Tampons allerdings nicht erreicht.

In der US 4,077,408 ist ein Tampon beschrieben, der aus einem in einer wasserlöslichen Hülle aufgenommenen Schaumkern besteht. Dadurch soll ein ausreichendes Ausdehnungsvermögen des Tampons während des Gebrauchs sichergestellt werden. Allerdings muß dazu erst die wasserlösliche Hülle aufgelöst werden, was wiederum zu Problemen hinsichtlich des Auslaufens von Körperflüssigkeit führen kann.

Ähnliche Tampons sind in der US 4,088,132 beschrieben. Auch diese Tampons sind im Hinblick auf die zuverlässige Vermeidung des Auslaufens von Körperflüssigkeit problematisch.

Endlich sind in der US 3,343,225 und US 3,515,138 Tampons nach dem Oberbegriff des Patentanspruchs 1 beschrieben, bei denen eine Vertiefung in der Kuppe des Tampons vorgesehen ist, um so die Oberfläche und das Saugvermögen des Tampons zu verbessern. Bei diesen bekannten Tampons ist der Übergang zwischen dem im wesentlichen zylinderförmigen Saugkörper und der Stirnfläche des Tampons abgerundet, wobei sich die trichterförmige Vertiefung in der Stirnfläche des Tampons im wesentlichen über die gesamte Stirnfläche bis zu dem abgerundeten Übergangsbereich erstreckt.

Es hat sich allerdings gezeigt, daß die mit diesen Tampons angestrebte Verbesserung des Saugvermögens mit der beschriebenen Tampongeometrie nicht erreicht werden kann und weiterhin das Auslaufen von Körperflüssigkeit aus der Körperhöhle beobachtet wird, selbst wenn das Absorptionsvermögen des Tampons noch nicht vollständig ausgenutzt ist.

Angesichts dieser Probleme im Stand der Technik liegt der Erfindung die Aufgabe zugrunde, eine Vorrichtung zum Herstellen von Tampons anzugeben, deren Absorptionsfähigkeit optimal ausgenutzt werden kann

Eine erfindungsgemäße Vorrichtung zur Herstellung eines Tampons mit einer Kuppenformeinrichtung zum Formen einer Kuppe an einem im wesentlichen zylindrischen und bereits gepreßten Tamponrohling, bei dem die Kuppenformeinrichtung mindestens ein Formwerkzeug mit einem beim Formvorgang in Richtung der Zylinderachse des Rohlings in den Rohling einführbaren Dorn aufweist, um so die das Einleiten der Menstruationsflüssigkeit in den Saugkörperkern begünstigende Vertiefung bzw. den entsprechenden trichterförmigen Einleitungskanal in der Tamponkuppe zu bilden, ist im wesentlichen dadurch gekennzeichnet, daß das Formwerkzeug einen ringförmigen Mantel mit sich zumindest abschnittweise in axialer Richtung verjüngendem Innendurchmesser und einem in den Mantel einsetzbaren Dorn aufweist.

Das erfindungsgemäße Formwerkzeug ist demnach modular aufgebaut. Dabei wird durch den Mantel die äußere konvexe und das Einführen des Tampons in die Körperhöhle begünstigende Oberfläche der Kuppe gebildet, während durch den in den Mantel eingesetzten Dorn die Vertiefung gebildet wird, mit der die Einleitung von Menstruationsflüssigkeit in den Saugkörperkern begünstigt wird. Durch den modularen Aufbau kann erreicht werden, daß bei einem mehrstufigen Herstellungsprozeß, bei dem der Saugkörper nacheinander in eine Anzahl von Werkzeugen eingesetzt wird unter Verwendung ein und desselben Mantels unterschiedliche Dornformen eingesetzt werden können. Bei dem beschriebenen modular aufgebauten Formwerkzeug kann der Erhalt der gewünschten Kuppenform besonders zuverlässig sichergestellt werden, wenn zwischen dem den sich verjüngenden Innendurchmesser aufweisenden Bereich der inneren Begrenzungsfläche des Mantels und dem Dorn ein Ringspalt freigelassen ist und der Mantelinnenraum an einem dem Dorn abgewandten axialen Ende mit einem vorzugsweise einstückig mit dem Dorn ausgeführten Dornträger verschlossen ist. Gegebenenfalls kann der Dorn in axialer Richtung über den dem Dornträger abgewandten Rand des Mantels hinausragen, um so eine die Kuppe vollständig durchsetzende und ggf. über die Kuppe hinausragende Vertiefung in der Kuppe zu bilden. Wie vorstehend bereits angedeutet, kann die Kuppenformeinrichtung zwei, drei oder mehr Formwerkzeuge aufweisen, in die die Rohlinge beim Formvorgang nacheinander einführbar sind.

Im Hinblick auf die Vereinfachung des Herstellungsvorgangs beim Einsatz mehrerer Formwerkzeuge sind diese zweckmäßigerweise an einem gemeinsamen Träger angeordnet, der bezüglich einer Drehachse drehbar gelagert ist, wobei die Formwerkzeug auf einer die Drehachse umlaufenden Kreislinie angeordnet sind. Dann können die Formwerkzeug durch Drehen des Trägers in dem Rohling gegenüberliegende Position gebracht werden und durch eine axiale Relativbewegung zwischen Rohling und Formwerkzeug der gewünschte Formgebungsvorgang eingeleitet werden.

Ein Formwerkzeug für eine erfindungsgemäße Vorrichtung ist im wesentlichen dadurch gekennzeichnet, daß es einen ringförmigen Mantel mit einem darin eingesetzten Dorn aufweist, wobei der Dorn zweckmäßigerweise austauschbar in dem Mantel aufgenommen ist. Ein erfindungsgemäßer Tampon wird, wie der vorstehenden Erläuterung zu entnehmen ist, hergestellt, in dem ein im wesentlichen zylindrischer und bereits radial und/oder linear gepreßter Tamponrohling in ein erfindungsgemäßes Formwerkzeug in axialer Richtung eingeführt wird.

Ein mit einer erfindungsgemäßen Vorrichtung hergestellter Tampon ist im wesentlichen dadurch gekennzeichnet, daß der Durchmesser der Vertiefung an ihrem dem Scheitelbereich zugewandten Rand etwa 10 bis 45 %, vorzugsweise etwa 20 bis 40 %, besonders bevorzugt etwa 25 bis 35 % des Saugkörperdurchmessers beträgt.

Dabei wird von der Erkenntnis Gebrauch gemacht, daß die mangelhaften Absorptionseigenschaften herkömmlicher Tampons darauf zurückzuführen sind, daß gerade im Kuppenbereich eine rasche Sättigung in den Randschichten des Tampons auftritt, die dann dazu führt, daß die Körperflüssigkeit am Tampon insgesamt vorbeiströmt und aus der zu verschließenden Körperhöhe ausläuft. Durch die erfindungsgemäße Weiterbildung wird im Kuppenbereich ein Trichter bzw. Saugkanal zur Verfügung gestellt, durch den die dort auftreffende Körperflüssigkeit, insbesondere Menstruationsflüssigkeit, direkt in den Saugkern des Saugkörpers gelangt, so daß eine frühzeitige Sättigung des äußeren Bereichs des Saugkörpers im Kuppenbereich zuverlässig verhindert werden kann und auch der innere Bereich des Tampons optimal genutzt werden kann. Dabei hat sich im besonderen gezeigt, daß durch die Bereitstellung der Vertiefung, d. h. eines konkaven Oberflächenbereichs im Bereich der ansonsten konvexen Tamponkuppe keine nennenswerten Beeinträchtigungen der Einführeigenschaften des Tampons, welche durch die Kuppe verbessert werden sollen, beobachtet werden. Insgesamt wird durch die erfindungsgemäße Weiterbildung von Tampons also die Absorptionseigenschaft des Tampons verbessert, ohne dessen sonstige Gebrauchseigenschaften zu beeinträchtigen.

Dabei ist der Durchmesser der Vertiefung an ihrem dem Scheitelbereich zugewandten Rand, d. h. der größte Durchmesser der Vertiefung in einer senkrecht zur Zylinderachse des Saugkörpers verlaufenden Richtung, so gewählt, daß er weniger als die Hälfte des Durchmessers des Saugkörpers beträgt. Diese Begrenzung des Vertiefungsdurchmessers geht auf die Erkenntnis zurück, daß die bei Tampons gemäß der US 3,343,225 beobachteten Probleme auf ein Zusammendrücken des Tampons im Bereich der Vertiefung zurückzuführen sind, wodurch die angestrebte Oberflächenvergrößerung und die gewünschte Verbesserung des Saugvermögens während des Gebrauchs verhindert wird. Wenn der Durchmesser der Vertiefung gemäß der vorliegenden Erfindung auf einen Wert von weniger als 50 % des Durchmessers des Saugkörpers eingestellt wird, kann eine ausreichende Stabilität des Tampons im Bereich der mit der Vertiefung versehenen Kuppe sichergestellt werden, so daß die angestrebte Oberflächenvergrößerung und die damit erreichte Verbesserung der Einleitung von Körperflüssigkeit in den Tamponkern zuverlässig erreicht wird, auch wenn der Tampon während des Gebrauchs einer Radialkraftbeaufschlagung unterzogen wird.

Falls der Durchmesser weniger als 20 % des Saugkörperdurchmessers beträgt, werden Beeinträchtigungen der gewünschten Eigenschaften hinsichtlich der Einleitung von Körperflüssigkeit in den Saugkern beobachtet. Bei einer Vergrößerung des Durchmessers der Vertiefung auf einen Wert von mehr als 40 % des Saugkörperdurchmessers kommt es zu Beeinträchtigungen bei der Einführung des Tampons in die Körperhöhle. Die trichterförmige Verjüngung der Vertiefung hat sich sowohl aus fertigungstechnischen Gründen als auch im Hinblick auf die gewünschte optimale Ausnutzung der Absorptionseigenschaften des Tampons als zweckmäßig erwiesen.

Aus fertigungstechnischer Sicht hat es sich als günstig erwiesen, wenn die erfindungsgemäß in der Tamponkuppe vorgesehene Vertiefung eine mindestens 2-zählige Drehsymmetrie bezüglich einer etwa kolinear zur Längsachse des Saugkörpers verlaufenden Symmetrieachse aufweist, also beispielsweise rechteckförmig ist. Im Hinblick auf eine gute Ausnutzung der Absorptionseigenschaften des Tampons ist es besonders zweckmäßig, wenn die Vertiefung eine 3-zählige Symmetrie mit dreieckförmigem Querschnitt, eine 4-zählige Symmetrie mit quadratischem Querschnitt und besonders bevorzugt eine Rotationssymmetrie mit kreisförmigem Querschnitt aufweist.

Im Hinblick auf die Optimierung der Einleitung von Körperflüssigkeit in den Saugkern des Saugkörpers hat es sich als zweckmäßig erwiesen, wenn sich die Vertiefung über mindestens 20 % insbesondere mindestens 50 % der axialen Länge der Kuppe erstreckt, die Kuppe vorzugsweise in axialer Richtung vollständig durchsetzt und besonders bevorzugt ausgehend von dem umlaufenden Rand über die Kuppe hinaus in den Saugkern hineinreicht. Dabei kann der Durchmesser der Vertiefung an ihrem die Zylinderachse umlaufenden Rand etwa 10 bis 45 %, insbesondere 20 bis 40 % , vorzugsweise etwa 25 bis 35 % des Saugkörperdurchmessers betragen.

Durch die Bereitstellung der Vertiefung in dem Tampon kann sich eine Verminderung der Menge absorptionsfähigen Materials des Tampons insgesamt ergeben. Im Hinblick auf die Optimierung der Ausnutzung der Absorptionsfähigkeit hat es sich als zweckmäßig erwiesen, wenn das Volumen der Vertiefung 4 % oder weniger, vorzugsweise 2 % oder weniger, besonders bevorzugt 1 % oder weniger, insbesondere 0,75 % oder weniger des Gesamtvolumens des Tampons entspricht. Dabei wird der gewünschte Effekt der Verbesserung der Einleitung von Körperflüssigkeit in den Tamponkern noch sichergestellt, wenn das Volumen der Vertiefung 0,1 % oder mehr, insbes. 0,2 % oder mehr, vorzugsweise 0,5 % oder mehr des Gesamtvolumens des Tampons entspricht.

Ähnlich wie bei herkömmlichen Tampons kann auch ein mit einer erfindungsgemäßen Vorrichtung hergestellter Tampon ein eine Mantelfläche des Saugkörpers abdeckendes Hüllmaterial, wie etwa ein auf Watte aufsiegelbares Hüllvlies oder eine vorzugsweise perforierte Hüllfolie, aufweisen, mit dem verhindert wird, daß Tamponfasern sich aus dem Tamponkörper lösen, wenn der Tampon sich im Rahmen der Aufnahme von Körperflüssigkeit ausdehnt. Im Rahmen der Erfindung einsetzbare Hüllmaterialien sind bspw. in der DE 69811114 T2, der EP 1194100 B1, der EP 925050 B1 und der US 68860874 beschrieben. Der Offenbarungsgehalt dieser Schriften wird hiermit durch ausdrückliche Inbezugnahme hinsichtlich der Beschaffenheit des Hüllmaterials in diese Beschreibung aufgenommen. Ferner können auch bei mit erfindungsgemäßen Vorrichtungen hergestellten Tampons zwei, drei oder mehr geradlinig, wellenförmig und/oder wendelförmig verlaufende Rillen in der Mantelfläche des Saugkörpers vorgesehen sein, um die Absorptionseigenschaften zu verbessern. Üblicherweise sind Tampons für den Transport und die Lagerung in einer Kunststofffolie aufgenommen. Dabei kommen regelmäßig Zellophanfolien zum Einsatz. Im Rahmen der Erfindung hat es sich gezeigt, daß das Ablösen von einzelnen Fasern im Bereich des Randes der Vertiefung bei dem Ablösen der Verpackungsfolie von dem Tampon gut verhindert werden kann, wenn anstelle üblicher Zellophanfolien eine OPP-Folie, also eine Polypropylenfolie zum Verpacken der Tampons eingesetzt wird.

Herkömmliche Tampons werden hergestellt, indem zunächst ein Hüllvlies auf ein Watteband aufgesiegelt wird, so daß das Hüllvlies sich über die Hälfte der Länge eines Wattebandstreifens erstreckt, der für die Herstellung eines Tampons benötigt wird und über den Wattestreifen hinausreicht. Beim nächsten Herstellungsschritt wird aus dem Wattestreifen ein Wattewickel gebildet, bei dem das Hüllvlies eine äußere Begrenzungsfläche des Wickels bildet, welcher den Wattestreifen überlappt und auf eine vorherige Wickellage des Hüllvlieses aufgesiegelt werden kann, um so eine umlaufende äußere Begrenzungsfläche des Wattewickels zu bilden. Anschließend wird der Wickel radial und/oder linear verpreßt, insbesondere um so einen etwa zylindrischen Saugkörper bereitzustellen. Im Rahmen der radialen Verpressung werden auch die ggf. vorzusehenden Rillen in der Mantelfläche des Saugkörpers gebildet. Zur Fertigstellung des Tampons wird der bereits gepreßte Tamponrohling in Formwerkzeuge einer Kuppenformeinrichtung eingeführt, deren Form der gewünschten Kuppenform entspricht.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert.

In der Zeichnung zeigt:
- **Fig. 1**: eine schematische Darstellung eines mit einer erfindungsgemäßen Vorrichtung hergestellten Tampons,
- **Fig. 2**: eine schematische Darstellung einer erfindungsgemäßen Vorrichtung zum Herstellen eines Tampons und
- **Fig. 3**: schematische Darstellungen von Formwerkzeugen einer erfindungsgemäßen Vorrichtung.

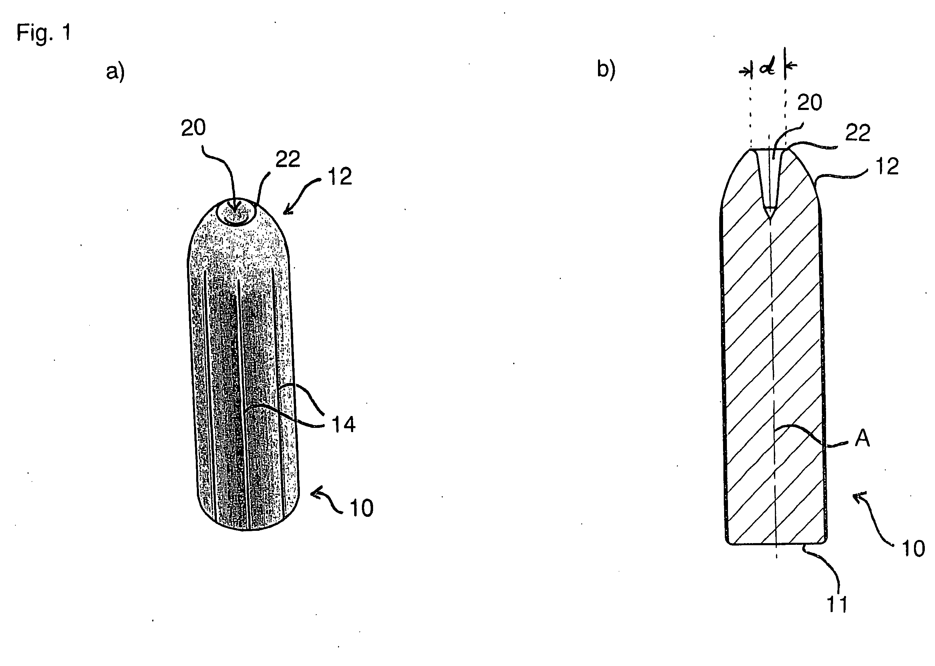

Gemäß Fig. 1 umfaßt der Tampon einen insgesamt mit 10 bezeichneten im wesentlichen zylindrischen Saugkörper, der an einem seiner axialen Enden eine sich in Richtung auf ein axiales Ende davon verjüngende Kuppe 12 aufweist. Der Saugkörper 10 weist in seiner Mantelfläche eine Anzahl von parallel zueinander und parallel zur Zylinderachse A des Saugkörpers verlaufenden Rillen 14 auf. Im Scheitelbereich der Kuppe 12 ist eine sich ausgehend von einem die Zylinderachse A umlaufenden Rand 22 in Richtung auf ein der Kuppe 12 abgewandtes axiales Ende 11 des Saugkörpers 10 erstreckende trichterförmige Vertiefung 20 gebildet, welche bei dem in der Zeichnung dargestellten Ausführungsbeispiel der Erfindung rotationssymmetrisch bezüglich der Zylinderachse A ausgeführt ist. Wie besonders deutlich der Fig. 1b zu entnehmen ist, verjüngt sich der Querschnitt der Vertiefung 20 ausgehend vom Rand 22 in Richtung auf das der Kuppe 11 abgewandte axiale Ende des Saugkörpers 10.

Der Durchmesser "d" der Vertiefung an ihrem dem Scheitelbereich zugewandten Rand (22), d. h. der größte Durchmesser der Vertiefung in einer sich senkrecht zur Zylinderachse "A" erstreckenden Richtung, beträgt bei der in der Zeichnung dargestellten Ausführungsform der Erfindung etwa 30 % des Saugkörperdurchmessers.

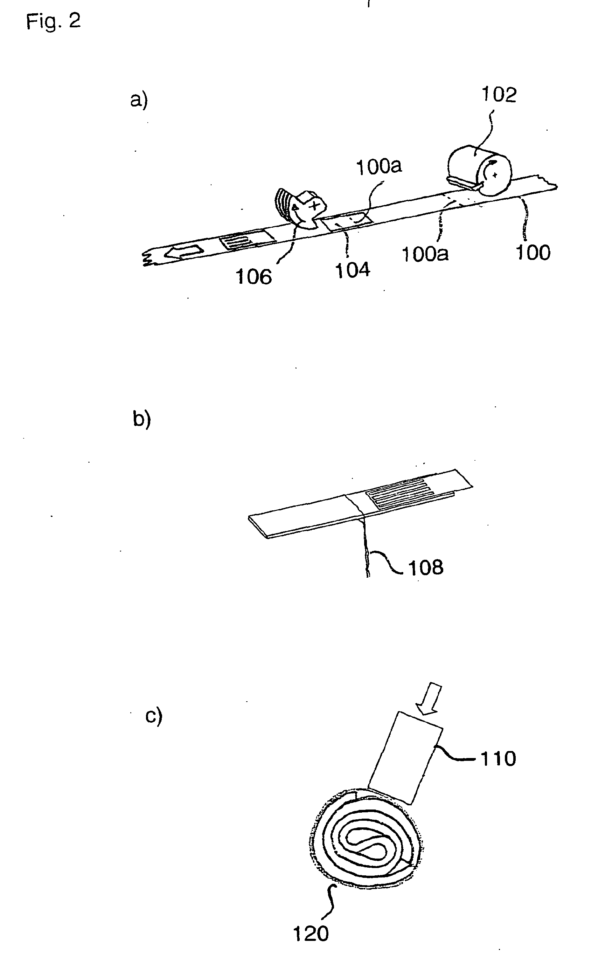

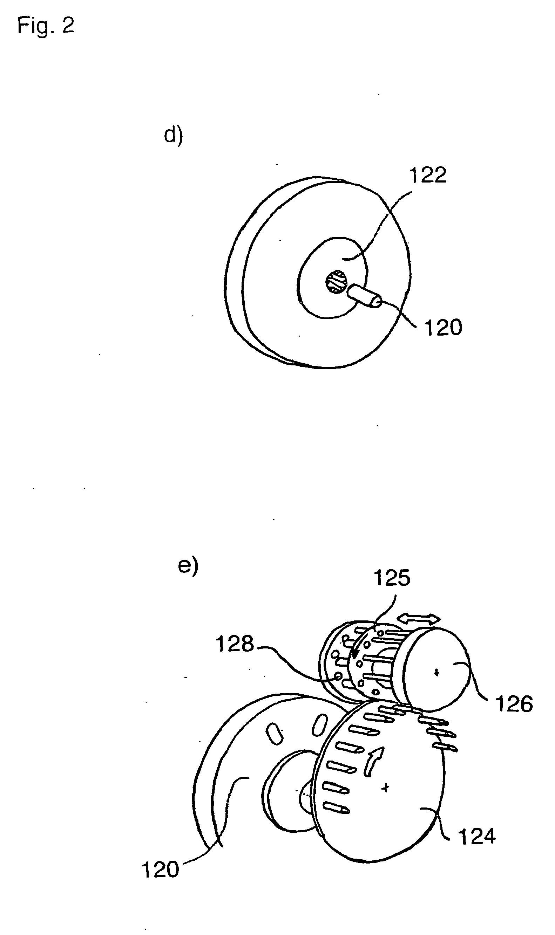

Tampons gemäß Fig. 1 können mit den nachstehend anhand der Fig. 2 erläuterten Vorrichtungen hergestellt werden. Gemäß Fig. 2 wird bei der Herstellung eines Tampons zunächst ein Watteband 100 in eine Tamponmaschine eingezogen und mit einem Wattemesser 102 auf die zur Herstellung einzelner Tampons benötigte Länge geschnitten, wie in Fig. 2a anhand der strichlinierten Linien 100a angedeutet.

Nach Schneiden des Wattebands zu Wattestreifen mit der benötigten Länge wird ein Hüllvlies 104 mit etwa der halben Länge der Wattestreifen versetzt um die halbe Länge eines Wattebandstreifens unter Druck und Hitze mit Hilfe einer Siegelbacke 106 auf das Watteband 100 aufgesiegelt. Das Hüllvlies ragt in Längsrichtung über den Wattestreifen hinaus, auf dem es aufgesiegelt ist. In einem nächsten Herstellungsschritt wird ein Rückholfaden 108 um den Wattestreifen 100 geschlungen und verknotet, wie in Fig. 2b dargestellt. Der Wattestreifen 100 wird zu einem sogenannten Wickel gerollt, derart daß der über den Wattestreifen hinausragende Bereich des Hüllvlies auf dem Wickel aufliegt und mit der vorhergehenden Hüllvlies-Wickellage versiegelt werden kann. Dazu wird eine Siegelbacke 110 eingesetzt, wie in Fig. 2c schematisch angedeutet.

Der so vorbereitete Wattewickel 120 wird an eine geöffnete Presse 122 übergeben, wie in Fig. 2d angedeutet. Durch Schließen der Presse 122 wird ein Rohling mit Rillen und entsprechendem Durchmesser (abhängig von der Größe, z. B. Mini, Normal, Super...) und anderen einstellbaren Parametern gepreßt.

Nach leichtem Anlüften der Presse 120 wird der damit erhaltene gepreßte und im wesentlichen zylinderförmige Rohling mit einem Ausschieber nach hinten aus der Presse gedrückt und an eine Röhrchentrommel 124 übergeben. In einer Kopftrommel 125 wird der Rohling mit Stößeln 126 in vorgegebener Reihenfolge in Formwerkzeuge einer Kuppenformeinrichtung gedrückt. Abhängig von der gewünschten Kuppenform und Ausführung des Tampons sind in der Kuppenformeinrichtung 128 4 bis 12 gleiche oder auch unterschiedliche Formwerkzeug eingebaut.

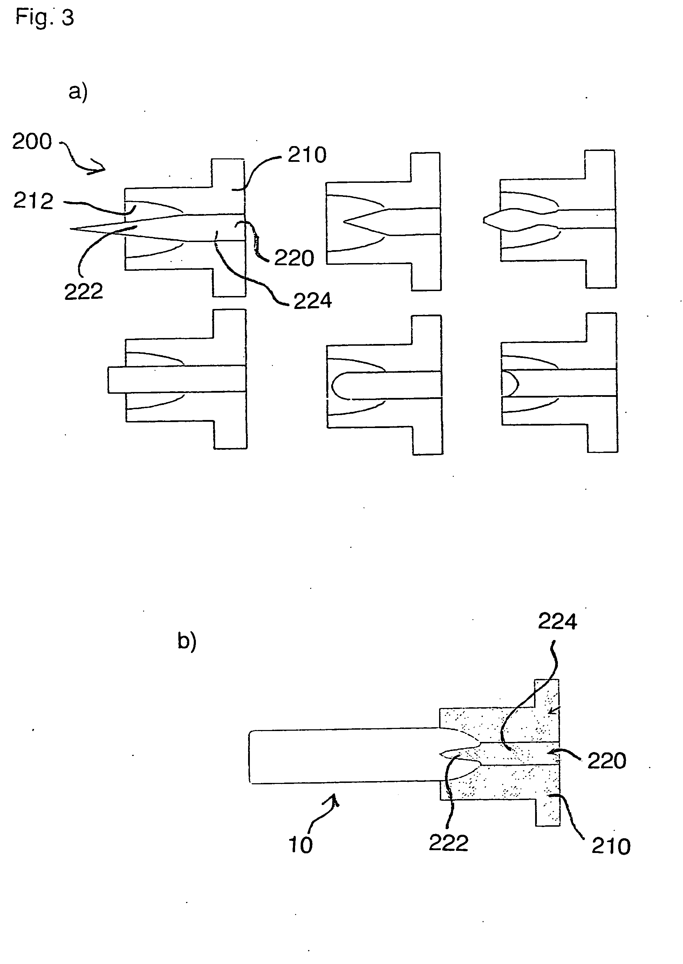

Die in einer erfindungsgemäßen Vorrichtung zur Herstellung von Tampons zum Einsatz kommenden Formwerkzeuge werden nachstehend anhand der Fig. 3 erläutert. Danach weisen die insgesamt mit 200 bezeichneten Formwerkzeuge einen ringförmigen Mantel 210 und eine Dornanordnung 220 auf. Die Dornanordnung besteht aus einem Dorn 222 und einem Dornträger 224. Der Mantel 210 weist eine innere Begrenzungsfläche 212 mit sich zumindest abschnittweise in axialer Richtung verjüngendem Innendurchmesser auf. Insgesamt ist der Mantel 210 rotationssymmetrisch ausgeführt. Der Dorn 222 ist derart in den Mantel 210 eingeführt, daß zwischen dem den sich verjüngenden Innendurchmesser enthaltenden Bereich der inneren Begrenzungsfläche 212 des Mantels und dem Dorn ein Ringspalt freigelassen ist und der Mantelinnenraum an seinem dem Dorn 222 abgewandten axialen Ende mit dem einstückig mit dem Dorn 222 gebildeten Dornträger 224 verschlossen ist. Gemäß Fig. 3a können bei Einsatz identisch ausgeführter Mantelelemente 210 unterschiedlich ausgeführte Dorne zum Einsatz kommen. Dabei können längere, kürze, dickere oder dünnere Dorne bzw. Dorne mit zylindrischem, bombiertem oder gewelltem Verlauf benutzt werden, wobei die Dornform entsprechend der gewünschten Form der Vertiefung 20 in der Kuppe des Tampons gewählt wird. Gemäß Fig. 3b wird der Tamponrohling in axialer Richtung in das Formwerkzeug 200 eingeführt, so daß der Dorn 222 in axialer Richtung in den Tampon eindringt, während gleichzeitig der äußere Kuppenring gebildet wird. Bei der in der Zeichnung dargestellten Ausführungsform der Erfindung beträgt das Volumen der Vertiefung ausgehend von dem umlaufenden Rand 22 bis zum der Kuppe 12 abgewandten axialen Ende 11 des Saugkörpers zugewandten Boden etwa 0,54 % des Gesamtvolumens des Tampons. Dabei kann sich der Durchmesser der Vertiefung ausgehend von einem Randdurchmesser im Bereich von etwa 5 mm ggf. über mehrere Stufen verjüngen und die Vertiefung in einer Spitze auslaufen, wie besonders deutlich in Fig. 1b zu erkennen ist, so daß ein in einen Kanal mündender Trichter gebildet wird, durch den Menstruationsflüssigkeit in den Kern des Saugkörpers 10 eingeleitet werden kann.

Die Erfindung ist nicht auf das anhand der Zeichnung erläuterte Ausführungsbeispiel beschränkt. Vielmehr ist auch daran gedacht, Tampons mit unterschiedlicher Form der Rillen 14 und/oder unterschiedlicher Querschnittsform der Vertiefung 20 einzusetzen. Es können Vertiefungen mit beliebigem, insbesondere dreieckigem, quadratischem oder rechteckigem Querschnitt zum Einsatz kommen. Bei dem anhand der Zeichnung erläuterten Ausführungsbeispiel der Erfindung durchsetzt die Vertiefung 20 die Kuppe 12 vollständig. Bei anderen Ausführungsformen der Erfindung kann die Vertiefung 20 über die Kuppe hinaus in den Saugkörperkern hineinreichen. In einigen Fällen ist es auch günstig, wenn die Vertiefung 20 nur einen Teil der Kuppe 12 durchsetzt. Zur Herstellung erfindungsgemäßer Tampons werden Tamponrohlinge in ein erfindungsgemäßes Formwerkzeug eingeführt. Dabei kann die Vertiefung in mehreren Schritten ausgeführt werden, indem der Tamponrohling nacheinander in identisch oder unterschiedlich ausgeführte Formwerkzeuge ausgeführt wird. Die Formwerkzeuge können dabei auf einer Formwerkzeugtrommel angeordnet sein, welche durch Drehen um eine Drehachse in eine dem Tampon gegenüberliegende Stellung gebracht wird, so daß der Tampon durch axiale Relativbewegung zwischen Formwerkzeug und Rohling nacheinander in unterschiedliche Formwerkzeuge eingeführt werden kann.

## Patentansprüche

1. Vorrichtung zur Herstellung eines Tampons zum Einführen in eine Körperhöhle mit einem im wesentlichen zylinderförmigen Saugkörper (10), der eine sich in Richtung auf ein axiales Ende verjüngende Kuppe (12) aufweist, bei dem in einem ein axiales Ende der Kuppe (12) bildenden Scheitelbereich davon eine sich ausgehend von einem die Zylinderachse (A) umlaufenden Rand davon in Richtung auf das der Kuppe (12) abgewandte Ende (11) des Saugkörpers (10) erstreckende und sich in Richtung auf das der Kuppe (12) abgewandte axiale Ende (11) des Saugkörpers (10) trichterförmig verjüngende Vertiefung (20) gebildet ist, deren Durchmesser an ihrem dem Scheitelbereich zugewandten Rand (22) etwa 10 bis 45 % des Saugkörperdurchmessers beträgt, mit einer Kuppenformeinrichtung (128) zum Formen einer Kuppe an einem im wesentlichen zylindrischen und bereits gepreßten Tamponrohling (120), bei dem die Kuppenformeinrichtung (128) mindestens ein Formwerkzeug (200) mit einem beim Formvorgang in Richtung der Zylinderachse des Rohlings (120) in den Rohling einführbaren Dorn (222) aufweist, **dadurch gekennzeichnet, daß** das Formwerkzeug (200) einen ringförmigen Mantel (210) mit sich zumindest abschnittweise in axialer Richtung verjüngenden Innendurchmesser und einem in den Mantel einsetzbaren Dorn (212) aufweist.

2. Vorrichtung nach Anspruch 1, **dadurch gekennzeichnet, daß** zwischen dem den sich verjüngenden Innendurchmesser aufweisenden Bereich (212) der inneren Begrenzungsfläche des Mantels (210) und dem Dorn (222) ein Ringspalt freigelassen ist und der Mantelinnenraum an seinem dem Dorn (222) abgewandten axialen Ende mit einem vorzugsweise einstückig mit dem Dorn (222) ausgeführten Dornträger (224) verschlossen ist.

3. Vorrichtung nach einem der Ansprüche 1 oder 2, **dadurch gekennzeichnet, daß** der Dorn (222) in axialer Richtung bündig mit dem Rand des Mantels (710) fluchtet.

4. Vorrichtung nach einem der vorhergehenden Ansprüche, **dadurch gekennzeichnet, daß** die Kuppenformeinrichtung (128) zwei, drei oder mehr Formwerkzeuge (200) aufweist, in die die Rohlinge (120) beim Formvorgang nacheinander einführbar sind.

5. Vorrichtung nach einem der vorhergehenden Ansprüche, **dadurch gekennzeichnet, daß** die Formwerkzeuge (200) an einem gemeinsamen Träger angeordnet sind.

6. Vorrichtung nach Anspruch 5, **dadurch gekennzeichnet, daß** die Kopfformtrommel bezüglich einer Drehachse drehbar ist und die Formwerkzeuge (200) auf einer die Drehachse umlaufenden Kreislinie angeordnet sind.

## Claims

1. A device for manufacturing a tampon for introduction into a body cavity comprising a substantially cylindrical suction body (10) which has a peak (12) tapered towards an axial end, wherein there is formed in an apex area forming an axial end of the peak (12) a recess (20) which extends starting from an edge surrounding the cylinder axis (A) towards the end (11) of the suction body (10) facing away from the peak (12) and tapering like a funnel towards the axial end (11) of the suction body (10) facing away from the peak (12), the diameter of said recess on its edge (22) facing the apex region being approximately 10 to 45 % of the suction body diameter, with a peak moulding device (128) for moulding a peak on a substantially cylindrical and already pressed tampon blank (120), wherein the peak moulding device (128) has at least one moulding tool (200) with a spur (222) that can be introduced in the direction of the cylinder axis of the blank (120) into the blank during the moulding process, **characterised in that** the moulding tool (200) has an annular jacket (210) with an internal diameter tapering at least in some sections in the axial direction and a spur (212) that can be inserted into the jacket.

2. The device according to Claim 1, **characterised in that** an annular gap is left free between the region (212) of the inner boundary surface of the jacket (210) and the spur (222) having the tapering internal diameter, and the interior of the jacket is closed on its axial end facing away from the spur (222) with a spur carrier (224) preferably made integrally with the spur (222).

3. The device according to either of Claims 1 or 2, **characterised in that** the spur (22) is aligned flush with the edge of the jacket (710) in the axial direction.

4. The device according to any of the preceding claims, **characterised in that** the peak moulding device (128) has two, three or more moulding tools (200) into which the blanks (12) can be introduced, one after the other, during the moulding process.

5. The device according to any of the preceding claims, **characterised in that** the moulding tools (200) are arranged on a common carrier.

6. The device according to Claim 5, **characterised in that** the head moulding drum is rotatable in relation to an axis of rotation, and the moulding tools (200) are arranged on a circular line surrounding the axis of rotation.

## Revendications

1. Dispositif de fabrication d'un tampon destiné à être introduit dans un orifice corporel présentant un corps absorbant (10) sensiblement cylindrique présentant une calotte (12) allant en rétrécissant dans la direction d'une extrémité axiale, dans lequel il est conçu, dans une partie de sommet de celui-ci constituant une extrémité axiale de la calotte (12), un renfoncement (20) qui s'étend depuis un bord du corps absorbant en entourant l'axe (A) du cylindre en direction de l'extrémité axiale (11) du corps absorbant (10) qui est opposée à la calotte, lequel renfoncement va en rétrécissant à la manière d'un entonnoir et présente un diamètre au niveau de son bord (22) tourné vers la partie de sommet faisant d'environ 10 à 45 % du diamètre du corps absorbant, comportant un dispositif de formage de calotte (128) destiné à former une calotte sur une ébauche de tampon (120) sensiblement cylindrique et préalablement pressée, le dispositif de formage de calotte (128) présentant au moins un outil de formage (200) comportant une broche (222) susceptible d'être introduite dans l'ébauche au cours du processus de formage, dans le sens de l'axe de cylindre de l'ébauche (120), **caractérisé en ce que** l'outil de formage (200) présente une jupe annulaire (210) possédant un diamètre intérieur allant en rétrécissant au moins par sections dans le sens axial ainsi qu'une broche (212) susceptible de s'insérer dans la jupe.

2. Dispositif selon la revendication 1, **caractérisé en ce qu'**un espace annulaire est laissé libre entre la zone (212) présentant le diamètre intérieur allant en rétrécissant sur la surface de délimitation intérieure de la jupe (210) et la broche (222), et **en ce que** l'espace intérieur de la jupe est fermé, au niveau de son extrémité axiale opposée à la broche (222), par un support de broche (224) conçu de préférence d'une seule pièce avec la broche (222).

3. Dispositif selon l'une des revendications 1 ou 2, **caractérisé en ce que** la broche (222) vient affleurer, dans le sens axial, avec le bord de la jupe (710).

4. Dispositif selon l'une des revendications précédentes, **caractérisé en ce que** le dispositif de formage de calotte (128) présente deux, trois ou plus de trois outils de formage (200) dans lesquels les ébauches (120) peuvent être introduites de manière successive au cours du processus de formage.

5. Dispositif selon l'une des revendications précédentes, **caractérisé en ce que** les outils de formage (200) sont agencés sur un support commun.

6. Dispositif selon la revendication 5, **caractérisé en ce que** le tambour de tête est rotatif par rapport à un axe de rotation et les outils de formage (200) sont agencés sur une ligne circulaire entourant l'axe de rotation.
